(19) **Europäisches Patentamt / European Patent Office / Office européen des brevets**

(11) **EP 3 135 119 B1**

(12) **EUROPEAN PATENT SPECIFICATION**

(45) Date of publication and mention
of the grant of the patent:
**06.03.2019 Bulletin 2019/10**

(51) Int Cl.:
*A23L 2/00* (2006.01)          *A23L 2/52* (2006.01)
*A23L 29/10* (2016.01)        *A23L 33/00* (2016.01)
*A23L 33/10* (2016.01)        *A23L 33/115* (2016.01)
*A61K 31/23* (2006.01)        *A23L 33/12* (2016.01)

(21) Application number: **16186275.0**

(22) Date of filing: **30.08.2016**

(54) **BEVERAGE COMPOSITION FOR WEIGHT GAIN SUPPRESSION**

GETRÄNKEZUSAMMENSETZUNG ZUR GEWICHTSZUNAHMEUNTERDRÜCKUNG

COMPOSITION DE BOISSON POUR LA SUPPRESSION DE PRISE DE POIDS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.08.2015 JP 2015170896**

(43) Date of publication of application:
**01.03.2017 Bulletin 2017/09**

(73) Proprietor: **Miyagi Health Innovation, Ltd.
Sendai-shi, Miyagi (JP)**

(72) Inventors:
• **HATAKEYAMA, Masaki
Miyagi, 9840075 (JP)**
• **ISAWA, Ryohei
Miyagi, 9840075 (JP)**

(74) Representative: **Hess, Peter K. G.
Bardehle Pagenberg Partnerschaft mbB
Patentanwälte, Rechtsanwälte
Prinzregentenplatz 7
81675 München (DE)**

(56) References cited:
**WO-A1-2009/005519       WO-A1-2009/118968
WO-A2-2015/127320       US-A1- 2004 142 018
US-A1- 2011 200 734**

• **MUMME KAREN ET AL: "Effects of Medium-Chain
Triglycerides on Weight Loss and Body
Composition: A Meta-Analysis of Randomized
Controlled Trials", JOURNAL OF THE ACADEMY
OF NUTRITION AND DIETETICS, vol. 115, no. 2, 1
February 2015 (2015-02-01), pages 249-263,
XP029191561, ISSN: 2212-2672, DOI:
10.1016/J.JAND.2014.10.022**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

TECHNICAL FIELD

The present invention relates to a use of a beverage composition having for a main ingredient thereof a medium chain fatty acid triglyceride, and more particularly, to a beverage composition used in an oil beverage having a high ketone ratio.

BACKGROUND ART

Medium chain fatty acid triglycerides are known biochemically to be more rapidly broken down following digestion and absorption than typical long chain fatty acid triglycerides, and subsequently converted to energy in the body without being stored as fat in the liver and other organs.
As a result of having these characteristics, medium chain fatty acid triglycerides are attracting attention as a lipid that can be efficiently metabolized into energy without placing a burden on the liver.
Historically, classic ketogenic diets containing mainly long chain fatty acids were used practically as a dietetic therapy for epilepsy around 1920. This epilepsy dietetic therapy was used to promote an increase in ketone body concentration in the blood by increasing the intake ratio of lipids (fatty acids) while strictly controlling the intake of other nutrients. Consequently, this dietetic therapy is also referred to as ketone dietetic therapy.
Subsequently, dietetic therapy for epilepsy went out of use following the development of other medications for epilepsy.
However, numerous research has demonstrated that reducing accumulation of fat in the liver and increasing ketone body concentration in the blood is not only effective for the treatment of epilepsy, but is also effective for ameliorating the lifestyle-related weight issues, and particularly obesity.
Here, what is important is the extent to which the intake ratio of lipids is increased in order to increase ketone body concentration in the blood for the purpose of ameliorating the lifestyle-related weight issues, and particularly obesity. Among today's lifestyles characterized by high ingestion of carbohydrates, in the case of examining ways to increase the intake ratio of lipids, it is preferable to ingest lipids in the form of medium chain fatty acid triglycerides that reduce the burden on the liver during metabolism.
However, medium chain fatty acid triglycerides are known to place a burden on the gastrointestinal tract when ingested in large amounts. Consequently, when medium chain fatty acid triglycerides were ingested in an amount that takes adequate advantage of their efficient energy metabolism properties, the burden on the gastrointestinal tract was excessively great, making it realistically difficult to ingest such large amounts.
In addition, although liquid diets and the like containing medium chain fatty acid triglycerides have been developed (see Patent Literature 1 below), the compositions of these liquid diets have yet to allow ingestion of an amount of medium chain fatty acid triglycerides that makes it possible to take adequate advantage of their efficient energy metabolism properties as described above.
Therefore, there was a desire to develop a beverage that is capable of increasing the intake ratio of medium chain fatty acid triglycerides, exhibiting low accumulation of fat in the liver, to a degree that efficiently promotes an increase in ketone body concentration in the blood while inhibiting a burden on the gastrointestinal tract. There was a particular desire to develop a medium chain fatty acid triglyceride-containing beverage having a high ketone ratio that can be readily ingested during the course of daily dietary life.

CITATION LIST

PATENT LITERATURE

Patent Literature 1

International Publication No. WO 2010/052847

US 2004/142018 relates to a regulator for the amount of body fat that can be used conveniently without any need for food restriction and any concern about the side effect of the regulator for the amount of body fat itself. WO 2009/118968 relates to a satiety inducing agent characterized by containing triglycerides containing medium-chain fatty acids in constituent fatty acids as an active ingredient.

SUMMARY OF THE INVENTION

An object of the present invention is to provide a use of a beverage composition for weight gain suppression containing as a main ingredient thereof an amount of medium chain fatty acid triglyceride that increases ketone body concentration in the blood, the beverage composition placing little burden on the gastrointestinal tract and being able to be realistically ingested as a part of daily dietary life.

As a result of conducting extensive studies in consideration of the above-mentioned circumstances, the inventors of the present invention discovered a beverage composition containing medium chain fatty acid triglyceride and an emulsifier, wherein each ingredient is incorporated so as to obtain a modified ketone ratio for which an increase in concentration of the ketone body fraction in the blood can be confirmed.

Namely, the beverage composition of the present invention is a beverage composition containing fatty acid triglyceride, emulsifier and carbohydrate; wherein, the fatty acid triglyceride contains a medium chain fatty acid triglyceride as a main ingredient, a medium chain fatty acid having 8 carbon atoms and a medium chain fatty acid having 10 carbon atoms contained as medium chain fatty acids that constitute the medium chain fatty acid triglyceride are such that the medium chain fatty acid having 8 carbon atoms is incorporated at 60 mass% or more and the medium chain fatty acid having 10 carbon atoms is incorporated at 40 mass% or less based on the total weight of the medium chain fatty acids, and the content of the fatty acid triglyceride is constituted so as to constitute 5.0 g to 11.0 g per 100 kcal of energy of the beverage composition.

In addition, the beverage composition of the present invention is constituted so that a modified ketone ratio calculated according to Equation 1 is 1.3 to 8.0.

[Equation 1]

$$\text{Modified ketone ratio} = \frac{0.9 \times F + 0.46 \times P}{Ca + 0.2 \times Cb + 0.1 \times F + 0.58 \times P}$$

(In Equation 1, F represents fat, P represents protein, Ca represents carbohydrate rapidly metabolized to glucose during the course of digestion and absorption, and Cb represents carbohydrates or saccharides not metabolized rapidly to glucose during the course of digestion and absorption, in terms of the weights thereof.)

Moreover, the beverage composition of the present invention is constituted so that the emulsifier constitutes 30 mg to 2000 mg per 100 kcal of energy of the beverage composition.

According to the present invention, use of a beverage composition is described, wherein the beverage composition can be provided that contains a large amount of medium chain fatty acid triglyceride, capable of efficient energy metabolism with little accumulation of fat in the liver, within a modified ketone ratio, and reduces the burden on the gastrointestinal tract by incorporating an emulsifier and the like.

In addition, the beverage composition of the present invention enables medium chain fatty acid triglyceride to be ingested readily during the course of daily dietary life when drunk since it employs a formulation that efficiently increases ketone body concentration in the blood while retaining the above-mentioned characteristics.

BRIEF DESCRIPTION OF THE DRAWINGS

FIG. 1 is a graph showing time-based changes in body weights of subjects in Example 3 in terms of the ratio with the initial weight of the subjects.

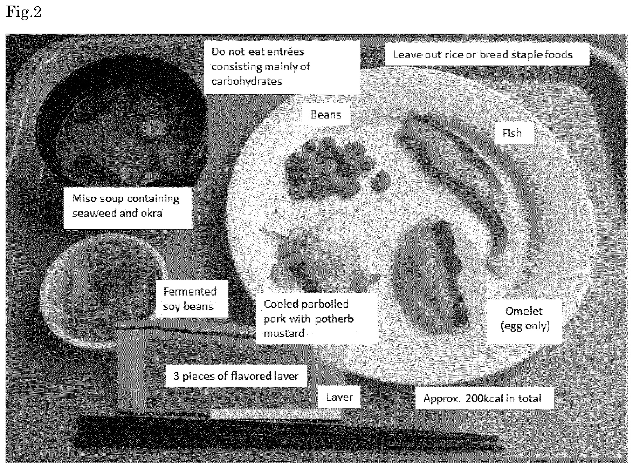

FIG. 2 is photograph showing an example of breakfast or lunch in Example 3.

DESCRIPTION OF EMBODIMENTS

The following provides a detailed explanation of embodiments of the present invention.

(Medium Chain Fatty Acid Triglyceride)

The beverage composition according to the use of the present invention contains a medium chain fatty acid triglyceride.

Medium chain fatty acids that constitute the medium chain fatty acid triglyceride consist of those having 6 carbon

atoms (caproic acid: $C_5H_{11}COOH$), those having 8 carbon atoms (caprylic acid: $C_7H_{15}COOH$), those having 10 carbon atoms (capric acid: $C_9H_{19}COOH$), and those having 12 carbon atoms (lauric acid: $C_{11}H_{23}COOH$). Among these, fatty acids having 8 carbon atoms that have a low coagulating point are incorporated at 60 mass% or more based on the total weight of medium chain fatty acids, while fatty acids having 10 carbon atoms are incorporated at 40 mass% or less based on the total weight of medium chain fatty acids from the viewpoint of having fatty acids of 8 carbon atoms and 10 carbon atoms as main components thereof, ease of maintaining the liquid form of a beverage at normal temperatures, and facilitating handling in the production process. Preferably fatty acids having 8 carbon atoms are incorporated at 70 mass% or more while fatty acids having 10 carbon atoms are incorporated at 30 mass% or less.

The medium chain fatty acid triglyceride of the present invention may be synthesized by esterifying the above-mentioned medium chain fatty acids or may be obtained by treating naturally-occurring fat and oil components, and these may also be used in combination.

The content of fatty acid triglyceride according to the use of the present invention is adjusted to 5.0 g to 11.0 g per 100 kcal of energy of the beverage composition so as to be an amount sufficient for promoting an increase in ketone body concentration in the blood by efficiently producing ketone bodies even when drunk supplementarily during the course of daily dietary life. If the content of fatty acid triglyceride is less than 5 g per 100 kcal of energy of the beverage composition, this leads to ingestion of a large amount of the beverage per se, including meals other than the beverage, in order to efficiently produce ketone bodies, thereby making this unrealistic. On the other hand, if the content of fatty acid triglyceride exceeds 11 g per 100 kcal of energy of the beverage composition, the beverage per se is perceived as an "oil" per se, thereby making it difficult to ingest. Consequently, the content of fatty acid triglyceride is adjusted to within the range of 5.0 g to 11.0 g per 100 kcal of energy of the beverage composition, and more preferably to 8.0 g to 11.0 g per 100 kcal of energy of the beverage composition.

(Fatty Acid Triglyceride)

The beverage composition according to the use of the present invention contains fatty acid triglyceride, and the fatty acid triglyceride may contain long chain fatty acid triglyceride in addition to the above-mentioned medium chain fatty acid triglyceride. The medium chain fatty acid triglycerides are incorporated at 80 mass% or more, preferably at 90 mass% or more, based on the total fatty acid triglycerides. If the content of the medium chain fatty acid triglycerides is less than 80 mass% based on the total fatty acid triglyceride, the beverage cannot efficiently promote an increase in ketone body concentration in the blood.

Examples of long chain fatty acids that constitute long chain fatty acid triglyceride include saturated and unsaturated fatty acids having 14 to 22 carbon atoms such as myristic acid, palmitic acid, stearic acid, palmitoleic acid, oleic acid, linoleic acid, $\alpha$-linolenic acid, $\gamma$-linolenic acid, eicosapentaenoic acid, docosahexanoic acid and the like.

The fatty acid triglyceride of the present invention may be contained as synthesized medium chain fatty acid triglyceride or long chain fatty acid triglyceride, or may be contained as edible oil.

Examples of edible oils include coconut oil, perilla oil (egoma oil), flaxseed oil, soybean oil, rapeseed oil and the like.

(Modified Ketone Ratio) The modified ketone ratio of the beverage composition according to the use of the present invention is adjusted to 1.3 to 8.0. The modified ketone ratio is calculated according to the following Equation 1.

[Equation. 1]

$$\text{Modified ketone ratio} = \frac{0.9 \times \text{F} + 0.46 \times \text{P}}{\text{Ca} + 0.2 \times \text{Cb} + 0.1 \times \text{F} + 0.58 \times \text{P}}$$

(In Equation 1, F represents fat, P represents protein, Ca represents carbohydrate rapidly metabolized to glucose during the course of digestion and absorption, and Cb represents carbohydrates or saccharides not metabolized rapidly to glucose during the course of digestion and absorption, in terms of the weights thereof.)

The reason for using this modified ketone ratio equation in the present invention is as indicated below. First, in the case of conventional ketone dietetic therapy, the degree of dietetic therapy is indicated with ketone ratio calculated according to Equation 2.

[Equation. 2]

$$\text{Ketone ratio} = \frac{0.9 \times F + 0.46 \times P}{C + 0.1 \times F + 0.58 \times P}$$

In Equation 2, F represents fat, P represents protein and C represents carbohydrate in terms of their respective weights.

Here, both ordinary carbohydrates such as rice and bread that are rapidly metabolized to glucose during the course of digestion and absorption, and carbohydrates such as erythritol or dietary fiber that are not rapidly metabolized to glucose, are included in the carbohydrates represented by C in Equation 2.
However, it is necessary to distinguish these carbohydrates since the main ingredient of fat in the present invention is medium chain fatty acid triglyceride for which there is little accumulation of fat in the liver.

Therefore, the inventors of the present invention decided to prepare the beverage composition by revising the conventional ketone ratio (Equation 2) to a modified ketone ratio (Equation 1).

In order to determine the lower limit of the modified ketone ratio calculated according to Equation 1, beverage compositions having different modified ketone ratios as shown in Table 1 were prepared and the modified ketone ratios of these beverage compositions were measured along with the concentrations of ketone body fractions in the blood.

[Table 1]

|  | Example 1 | Comparative Example 1 |
| --- | --- | --- |
| Fat (Medium chain fatty acid triglyceride) | 13g | 10g |
| Fat (Long chain fatty acid triglyceride) | 1.21g | 1.99g |
| Protein | 4.31g | 4.99g |
| Carbohydrate | 5.47g | 6.46g |
| Emulsifier | 120mg | 120mg |
| Modified ketone ratio | 1.57 | 1.24 |

Subjects ingested the beverage composition of Example 1 (modified ketone ratio: 1.57) on three occasions consisting of before bed after ingesting an evening meal (normal diet) on the day before testing, after breakfast (light normal diet lowered to 200 kcal) on the day of testing, and before lunch on the day of testing. Blood samples were collected 1 to 2 hours after ingesting the beverage composition of Example 1 before lunch followed by investigation of the blood concentration of the ketone body fraction of venous blood serum (using an enzymatic method).

The reason for collecting blood samples 1 to 2 hours after ingestion of the beverage composition before lunch is as explained below. Medium chain fatty acid triglycerides are known to be rapidly broken down in the liver following digestion and absorption resulting in a rise in ketone body concentration in the blood. Since it was surmised on the basis of extensive research conducted by the inventors of the present invention that energy is stored in the body following ingestion of a meal containing medium chain fatty acid triglycerides roughly 2 hours after eating based on secretion of insulin and changes in blood sugar levels, blood samples were collected 1 to 2 hours after ingestion before lunch. Furthermore, surviving ketone dietetic therapy theory does not consider these time-based changes in ketone body concentrations in the blood immediately after eating, and states that it is necessary to maintain total dietary ketone ratio at 2.0 or more.

A similar investigation was carried out for Comparative Example 1 (modified ketone ratio: 1.24) on a different day.

The results are shown in Table 2.

[Table 2]

| Blood Concentration of Ketone Body Fraction in Venous Blood Serum | | | | | | | |
|---|---|---|---|---|---|---|---|
| | | Unit | Subject 1 | Subject 2 | Subject 3 | Average | |
| Example 1 ingestion group | Total ketone bodies | μmol/L | 699 | 307 | 798 | 601 | |
| | Acetoacetic acid | μmol/L | 559 | 69 | 132 | 253 | |
| | 3-hydroxybutyric acid | μmol/L | 140 | 238 | 666 | 348 | |
| | | Unit | Subject 1 | Subject 2 | Subject 4 | Subject 5 | Average |
| Comparative Example 1 ingestion group | Total ketone bodies | μmol/L | 88 | 130 | 193 | 207 | 155 |
| | Acetoacetic acid | μmol/L | 27 | 43 | 51 | 51 | 43 |
| | 3-hydroxybutyric acid | μmol/L | 61 | 87 | 142 | 156 | 112 |

Furthermore, the normal values (reference) for blood concentrations of the ketone body fractions of venous blood serum are 131μmol/L or less for total ketone bodies, 55μmol/L or less for acetoacetic acid, and 85μmol/L or less for 3-hydroxybutyric acid.

As shown in Table 2, in contrast to the blood concentrations of the ketone body fraction in the Comparative Example 1 ingestion group being somewhat higher than the reference normal values, blood concentrations of the ketone body fraction in the Example 1 ingestion group were confirmed to be more than 4 times higher.

Accordingly, the lower limit value of the modified ketone ratio of the beverage composition of the present invention was set to 1.3.

Furthermore, if the modified ketone ratio of the beverage composition exceeds 8.0, the beverage per se is perceived as an "oil" per se, thereby making it difficult to ingest. Consequently, the modified ketone ratio is adjusted to 1.3 to 8.0 as previously described.

(Emulsifier)

Ingestion of medium chain fatty acid triglyceride only, namely ingestion of a beverage consisting of an oil only, is difficult from the viewpoints of flavor and personal preference. Consequently, there are typically many cases in which medium chain fatty acid triglycerides are mixed with salad dressing or used in cooking.

However, in the case of using in such as a manner or amount, efficient energy metabolism of a large amount of medium chain fatty acid triglyceride within the range of the modified ketone ratio is not possible, thereby preventing the object of the present invention from being achieved.

Therefore, an emulsifier should be incorporated in the beverage composition of the present invention.

The emulsifier used in the present invention is incorporated as a beverage, and there are no particular limitations thereon provided it is able to adequately emulsify the main ingredient in the form of the medium chain fatty acid triglyceride. Examples of emulsifiers include lecithin, polyglycerin fatty acid esters, decaglycerin fatty acid esters, sorbitol fatty acid esters, succinic acid esters of monoglyceride and the like.

Among these, lecithin, which has the same composition as human cell membranes, is safer, and can be ingested in large amounts, and decaglycerin fatty acid esters or polyglycerin fatty acid esters, which facilitate handling when incorporating into water-soluble drinks in the production process, are more preferable.

In examining the content of emulsifier, since the medium chain fatty acid triglyceride of the present invention places a burden on the gastrointestinal tract while demonstrating little storage of fat in the liver as previously described, it is required to be adequately emulsified with an emulsifier.

Therefore, in order to determine the lower limit of emulsifier content, beverage compositions having different emulsifier incorporation ratios were prepared as shown in Table 3 followed by carrying out a sensory test on the gastrointestinal tract.

[Table 3]

| | Example 2 | Comparative Example 2 |
|---|---|---|
| Fat (Medium chain fatty acid triglyceride) | 20g | 20g |
| Milk | 30ml | 30ml |

(continued)

|  | Example 2 | Comparative Example 2 |
|---|---|---|
| Protein | 3g | 3g |
| Emulsifier | 120mg | 40mg |
| *Emulsifier (per 100kcal) | 56.3mg | 18.8mg |

Decaglycerin stearic acid ester was used for the emulsifier in Table 3.

Eleven subjects were allowed to eat breakfast (normal diet) and lunch (normal diet) on the day of the sensory test, the beverage composition of Example 2 was ingested at 14:00 after lunch, and the beverage composition of Example 2 was subsequently ingested at 18:00 without ingesting any other foods or drinks. Symptoms were then investigated starting 30 minutes to 2 hours later.

A similar test was carried out for the beverage composition of Comparative Example 2 on a different day.

The results are shown in Table 4.

[Table 4]

| Sensory Test | | |
|---|---|---|
|  | Example 2 | Comparative Example 2 |
| Emulsifier content (per 100kcal) | 56.3mg | 18.8mg |
|  | (Out of 11 people, Multiple answers possible) | (Out of 11 people, Multiple answers possible) |
| Stomachache | 1 | 1 |
| Discomfort in the stomach | 1 | 1 |
| Nausea/vomiting | 0 | 0 |
| Sensation of heat in abdomen | 0 | 0 |
| Discomfort caused by increased gurgling sounds | 0 | 4 |
| Discomfort caused by diarrhea/soft stools | 0 | 2 |

As shown in Table 4, in contrast to subjects who ingested the beverage composition of Comparative Example 2 complaining of stomachache, discomfort in the stomach, discomfort caused by increased gurgling sounds, and discomfort caused by diarrhea or soft stools, in the case of the beverage composition of Example 2, although there were some subjects who complained of stomachache and discomfort in the stomach, the number of subjects who complained of discomfort clearly decreased overall.

On the basis of the above results of the sensory tests and in consideration of fluctuations in the data, the lower limit of the content of the emulsifier of the present invention was set to 30 mg.

Furthermore, although a higher content of emulsifier theoretically results in a greater decrease in the burden on the gastrointestinal tract attributable to medium chain fatty acid triglyceride, since the emulsifier per se has a bitter taste and causes a heavy sensation, the incorporation of a large amount is not realistic in terms of personal preference.

Consequently, the content of emulsifier is adjusted to 30 mg to 2000 mg per 100 kcal of energy of the beverage composition as previously described.

(Other Ingredients)

The beverage composition according to the use the present invention may be made into the form of a beverage by adding ingredients capable of being incorporated in beverages within a range that does not impair the effects of the present invention. Examples of other ingredients include vitamin C, acidifiers, aromatics, sweeteners, erythritol, galactooligosaccharides, water and the like.

(Ingestion Method)

The beverage composition according to the use of the present invention can be used so as to ingest the medium chain fatty acid triglyceride of 50 g to 180 g (450 kcal to 1620 kcal) per day together with ingestion of daily meals of 250 kcal to 700 kcal per day in order to promote the efficient energy metabolism of the medium chain fatty acid triglyceride contained therein.

The above-mentioned daily meals refer to meals consisting of foods that are routinely ingested on a daily basis, and preferably refer to meals containing a proper balance of necessary nutrients. The ingested amount of these daily meals is held to a lower amount than the ordinary ingested amount in order to ingest together with the beverage composition according to the use of the present invention. In addition, the amount of carbohydrates contained in the above-mentioned daily meals is more preferably reduced to the range of 40 g to 130 g in order to effectively increase ketone body concentration in the blood.

[Examples]

The composition of the beverage used in Example 3 (1 bottle: amount ingested per single use) is shown in Table 5.

[Table 5]

|  | Example 3 |
|---|---|
| Medium chain fatty acid triglyceride (C8:C10=75:25) | 25g |
| Processed soy milk | 50ml |
| Protein | 3g |
| Lecithin | 81mg |
|  |  |
| Total energy | 266kcal |
| Modified ketone ratio | 4.03 |

Eighteen subjects ingested the beverages and daily meals described in Example 3 according to the ingestion method described in Table 6 and body weights were measured at the same time each day (such as upon waking up in the morning and after bathing at night).

[Table 6]

| Time (example) | Ingested foods |
|---|---|
| 7:00 | Fruit and vegetable iuice 200ml (Carbohydrate 15g, Potassium approx. 800mg) |
| 8:00 | Entrée (ordinary reduced carbohydrate food: carbohydrate content: 15 g or less: 100-200kcal) |
| 8:15 | Supplement (containing 388 mg of lecithin) |
| 8:30 | Drink described in Example 3 |
| 11:00 | Fruit and vegetable juice 200ml (Carbohydrate 15g, Potassium approx. 800mg) |
| 12:00 | Entrée (ordinary reduced carbohydrate food: carbohydrate content: 15 g or less: 100-200kcal) |
| 12:15 | Supplement (containing 388 mg of lecithin) |
| 12:30 | Drink described in Example 3 |
| 18:00 | Entrée (one dish only) |
| 18:15 | Supplement (containing 388 mg of lecithin) |
| 18:30 | Drink described in Example 3 |
| 22:15 | Drink described in Example 3 containing 15 g of medium chain fatty acid triglyceride + 10-15ml of perilla oil |

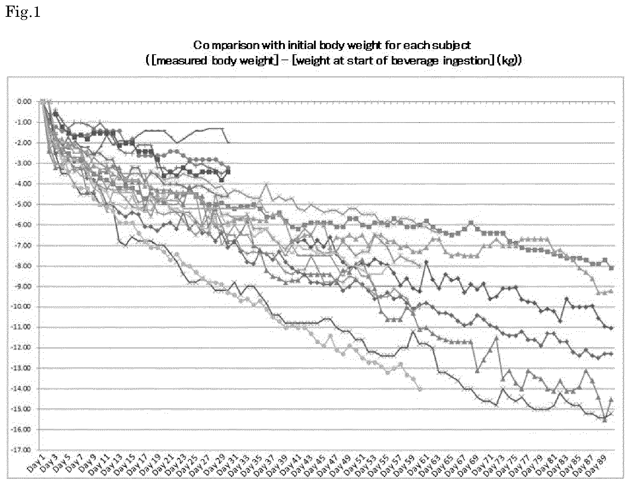

Time-based changes in body weights of the subjects are indicated in FIG. 1 in terms of the ratio with the initial body weight of that subject based on body weight at the start of ingestion of the beverage of Example 3.
In addition, body weight and other data of the subjects are shown in Table 7.

[Table 7]

| Subjects | Starting age | Gender | Test period (days) | Height (m) | Initial body weight (kg) | Ending body weight (kg) | Change (kg) | Rate of change in body weight (%) | Initial BMI | Ending BMI |
|---|---|---|---|---|---|---|---|---|---|---|
| Subject A | 55 | M | 153 | 1.700 | 103.60 | 79.50 | -24.10 | -23.26 | 35.8 | 27.5 |
| Subject B | 51 | M | 151 | 1.815 | 119.00 | 99.70 | -19.30 | -16.22 | 36.1 | 30.3 |
| Subject C | 45 | M | 158 | 1.700 | 91.50 | 78.50 | -13.00 | -14.21 | 31.7 | 27.2 |
| Subject D | 47 | M | 60 | 1.690 | 99.40 | 85.40 | -14.00 | -14.08 | 34.8 | 29.9 |
| Subject E | 35 | M | 60 | 1.670 | 72.50 | 62.50 | -10.00 | -13.79 | 26.0 | 22.4 |
| Subject F | 53 | M | 90 | 1.700 | 94.60 | 82.30 | -12.30 | -13.00 | 32.7 | 28.5 |
| Subject G | 41 | F | 90 | 1.480 | 63.70 | 55.60 | -8.10 | -12.72 | 29.1 | 25.4 |
| Subject H | 36 | F | 63 | 1.619 | 59.30 | 52.90 | -6.40 | -10.79 | 22.6 | 202 |
| Subject I | 44 | M | 90 | 1.740 | 90.00 | 80.80 | -9.20 | -10.22 | 29.7 | 26.7 |
| Subject J | 39 | M | 60 | 1.680 | 79.00 | 71.00 | -8.00 | -10.13 | 28.0 | 252 |
| Subject K | 34 | F | 57 | 1.650 | 91.60 | 83.60 | -8.00 | -8.73 | 33.6 | 30.7 |
| Subject L | 37 | M | 56 | 1.710 | 115.50 | 106.50 | -9.00 | -7.79 | 39.5 | 36.4 |
| Subject M | 36 | F | 30 | 1.580 | 65.80 | 61.20 | -4.60 | -6.99 | 26.4 | 24.5 |
| Subject N | 32 | F | 30 | 1.560 | 58.70 | 55.10 | -3.60 | -6.13 | 24.1 | 22.6 |
| Subject O | 34 | F | 30 | 1.550 | 59.20 | 56.00 | -3.20 | -5.41 | 24.6 | 23.3 |
| Subject P | 41 | F | 30 | 1.560 | 55.40 | 53.40 | -2.00 | -3.61 | 22.8 | 21.9 |
| Subject Q | 38 | M | 30 | 1.700 | 65.80 | 63.60 | -2.20 | -3.34 | 22.8 | 22.0 |
| Subject R | 44 | F | 30 | 1.635 | 54.60 | 53.00 | -1.60 | -2.93 | 20.4 | 19.8 |

All 18 subjects were confirmed to have lost weight in comparison with initial body weight at the start of ingestion of the beverage described in Example 3. The amount of that weight loss during the first month was an average of -6.8%.

The subject observed to lose the most weight demonstrated weight loss of -15.2 kg (119.0 kg -> 103.8 kg) over a period of three months, representing a decrease of -12.8% in comparison with initial body weight.

Furthermore, the study of the present example was started sequentially from September 2014 to June 2015. In addition, the study of the present example was carried out while confirming the health status of the subjects by a physician, and the health status of all subjects was confirmed to be free of any problems.

As indicated by Examples 1 to 3 described in the section on preferred embodiments of the present invention, despite the beverage composition according to the use of the present invention having for a main ingredient thereof an oil such as a medium chain fatty acid triglyceride, it was determined to be able to be readily ingested without causing a sense of hunger together with daily meals or daily meals having somewhat reduced carbohydrate levels, increase ketone body concentration in the blood while placing little burden on the gastrointestinal tract, and inhibit weight gain or reduce body weight, while also having the effect of decreasing BMI.

INDUSTRIAL APPLICABILITY

The beverage composition according to the use of the present invention is a medium chain fatty acid triglyceride having a high modified ketone ratio, and can be realistically ingested as a part of daily dietary life while placing little burden on the gastrointestinal tract. As a result of having such characteristics, it is able to effectively increase ketone body concentration in the blood by, for example, ingesting in combination with lower carbohydrate diets.

In particular, in light of the current growing size of the obese population, use of the beverage composition according to the present invention can be applied to suppress weight gain, and the use can be incorporated in daily life.

**Claims**

1. Use of a beverage composition for the non- therapeutic suppression of weight gain comprising, as active ingredients, fatty acid triglyceride, emulsifier and carbohydrate, **characterized in that**
   said fatty acid triglyceride contains a medium chain fatty acid triglyceride,
   a content of said medium chain fatty acid triglyceride is 80 mass% or more of said fatty acid triglyceride,
   medium chain fatty acid having 8 carbon atoms and medium chain fatty acid having 10 carbon atoms contained as medium chain fatty acids that constitute said medium chain fatty acid triglyceride are such that said medium chain fatty acid having 8 carbon atoms is incorporated at 60 mass% or more and said medium chain fatty acids having 10 carbon atoms is incorporated at 40 mass% or less based on the total weight of said medium chain fatty acids,
   a content of said fatty acid triglyceride is 5.0 g to 11.0 g per 100 kcal of energy of said beverage composition for weight gain suppression, and
   a modified ketone ratio calculated according to Equation 1 is 1.3 to 8.0

$$[\text{Equation. 1}]$$

$$\text{Modified ketone ratio} = \frac{0.9 \times F + 0.46 \times P}{Ca + 0.2 \times Cb + 0.1 \times F + 0.58 \times P}$$

(in Equation. 1, F represents fat, P represents protein, Ca represents carbohydrate rapidly metabolized to glucose during the course of digestion and absorption, and Cb represents carbohydrates or saccharides not metabolized rapidly to glucose during the course of digestion and absorption, in terms of the weights thereof),
   wherein said medium chain fatty acid triglyceride in the beverage composition for weight gain suppression is ingested at 50 g to 180 g (450 kcal to 1620 kcal) per day together with ingestion of a daily meal of 250 kcal to 700 kcal per day.

2. Use of the beverage composition according to claim 1, **characterized in that** said emulsifier is 30 mg to 2000 mg per 100 kcal of energy of said beverage composition for weight gain suppression.

3. Use of the beverage composition according to claim 1, **characterized in that** said emulsifier is lecithin or glycerin fatty acid ester.

4. Use of the beverage composition according to claim 1, **characterized in that** carbohydrate contained in said daily meal of 250 kcal to 700 kcal per day is 40 g to 130 g.

**Patentansprüche**

1. Verwendung einer Getränkezusammensetzung zur nicht-therapeutischen Unterdrückung von Gewichtszunahme, umfassend, als aktive Inhaltsstoffe, Fettsäure-Triglycerid, Emulgator und Kohlenhydrat, **dadurch gekennzeichnet, dass** das Fettsäure-Triglycerid ein mittelkettiges Fettsäure-Triglycerid beinhaltet, wobei ein Gehalt des mittelkettigen Fettsäure-Triglycerids 80 Masse-% oder mehr von dem Fettsäure-Triglyzerid ist,
wobei die mittelkettige Fettsäure, welche 8 Kohlenstoffatome aufweist und die mittelkettige Fettsäure, welche 10 Kohlenstoffatome aufweist, welche als mittelkettige Fettsäuren enthalten sind, welche das mittelkettige Fettsäure-Triglyzerid ausmachen, in solch einer Weise vorliegen, dass die mittelkettige Fettsäure, welche 8 Kohlenstoffatome aufweist, zu 60 Masse-% oder mehr enthalten ist und die mittelkettigen Fettsäuren, welche 10 Kohlenstoffatome aufweisen, zu 40 Masse-% oder weniger enthalten sind, basierend auf dem Gesamtgewicht der mittelkettigen Fettsäuren,
ein Gehalt von Fettsäure-Triglyceriden 5,0 g bis 11,0 g pro 100 kcal Energie der Getränkezusammensetzung zur Unterdrückung von Gewichtszunahme ist und ein modifiziertes Ketonverhältnis, welches gemäß Gleichung 1 berechnet wird, 1,3 bis 8,0 ist

[Gleichung 1]

$$\text{Modifiziertes Ketonverhältnis} = \frac{0{,}9 \times F + 0{,}46 \times P}{Ca + 0{,}2 \times Cb + 0{,}1 \times F + 0{,}58 \times P}$$

(in Gleichung 1 bezeichnet F Fett, P bezeichnet Protein, Ca bezeichnet Kohlenhydrat, welches schnell zu Glukose metabolisiert wird im Laufe der Verdauung und Absorption und Cb bezeichnet Kohlenhydrate oder Saccharide, welche nicht schnell zu Glukose metabolisiert werden im Laufe der Verdauung und Absorption, in Bezug auf die Gewichte davon),
wobei das mittelkettige Fettsäure-Triglyzerid in der Getränkezusammensetzung zur Unterdrückung von Gewichtszunahme eingenommen wird mit 50 g bis 180 g (450 kcal bis 1620 kcal) pro Tag, zusammen mit der Einnahme einer täglichen Mahlzeit von 250 kcal bis 700 kcal pro Tag.

2. Verwendung der Getränkezusammensetzung gemäß Anspruch 1, **dadurch gekennzeichnet, dass** der Emulgator 30 mg bis 2000 mg pro 100 kcal Energie der Getränkezusammensetzung zur Unterdrückung von Gewichtszunahme ist.

3. Verwendung der Getränkezusammensetzung gemäß Anspruch 1, **dadurch gekennzeichnet, dass** der Emulgator Lecithin oder Glycerinfettsäureester ist.

4. Verwendung der Getränkezusammensetzung gemäß Anspruch 1, **dadurch gekennzeichnet, dass** Kohlenhydrat, welches in der täglichen Mahlzeit von 250 kcal bis 700 kcal pro Tag mit 40 g bis 130 g enthalten ist.

**Revendications**

1. Utilisation d'une composition de boisson pour la suppression non thérapeutique de la prise de poids, comprenant, en tant que matières actives, un triglycéride d'acide gras, un émulsifiant et un glucide, **caractérisée en ce que** ledit triglycéride d'acide gras contient un triglycéride d'acide gras à longueur de chaine moyenne, une teneur en ledit triglycéride d'acide gras à longueur de chaine moyenne est de 80 % en masse ou plus dudit triglycéride d'acide gras,
un acide gras à longueur de chaine moyenne ayant 8 atomes de carbone et un acide gras à longueur de chaine moyenne ayant 10 atomes de carbone, contenus en tant qu'acides gras à longueur de chaine moyenne qui constituent ledit triglycéride d'acide gras à longueur de chaine moyenne, sont tels que ledit acide gras à longueur de chaine moyenne ayant 8 atomes de carbone est incorporé à raison de 60 % en masse ou plus et ledit acide gras à longueur de chaine moyenne ayant 10 atomes de carbone est incorporé à raison de 40 % en masse ou moins, par rapport au poids total desdits acides gras à longueur de chaine moyenne,
une teneur en ledit triglycéride d'acide gras est de 5,0 g à 11,0 g pour 100 kcal d'énergie de ladite composition de boisson pour la suppression de la prise de poids, et
un rapport cétonique modifié calculé selon l'Équation 1 est de 1,3 à 8,0

$$\text{ratio cétonique modifié} = \frac{0,9 \times F + 0,46 \times P}{Ca + 0,2 \times Cb + 0,1 \times F + 0,58 \times P} \qquad [\text{Équation 1}]$$

(dans l'Équation 1, F représente les lipides, P représente les protéines, Ca représente les glucides rapidement métabolisés en glucose au cours de la digestion et de l'absorption, et Cb représente les glucides ou les saccharides non métabolisés rapidement en glucose au cours de la digestion et de l'absorption, tous ces paramètres étant en poids),
ledit triglycéride d'acide gras à longueur de chaine moyenne présent dans la composition de boisson pour la suppression de la prise de poids étant ingéré à raison de 50 g à 180 g (450 kcal à 1620 kcal) par jour, en même temps que l'ingestion d'un repas journalier de 250 kcal à 700 kcal par jour.

2. Utilisation de la composition de boisson selon la revendication 1, **caractérisée en ce que** ledit émulsifiant correspond à 30 mg à 2000 mg par 100 kcal d'énergie de ladite composition de boisson pour la suppression de la prise de poids.

3. Utilisation de la composition de boisson selon la revendication 1, **caractérisée en ce que** ledit émulsifiant est la lécithine ou un ester d'acide gras du glycérol.

4. Utilisation de la composition de boisson selon la revendication 1, **caractérisée en ce que** le glucide contenu dans ledit repas journalier de 250 kcal à 700 kcal par jour est présent en une quantité de 40 g à 130 g.

Fig.1

**Comparison with initial body weight for each subject**
**([measured body weight] − [weight at start of beverage ingestion] (kg))**

Fig.2

**EP 3 135 119 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004142018 A

- WO 2009118968 A